# EUROPÄISCHE PATENTSCHRIFT

(11) **EP 4 618 909 B1**
(45) Veröffentlichungstag und Bekanntmachung des Hinweises auf die Patenterteilung: **06.05.2026**
(21) Anmeldenummer: 23801761.0
(22) Anmeldetag: 07.11.2023
(51) Int. Cl.: A61F 2/50, A61F 2/70, A61F 5/01

(54) **ORTHOPÄDIETECHNISCHE EINRICHTUNG**
ORTHOPAEDIC TECHNICAL DEVICE
DISPOSITIF TECHNIQUE ORTHOPÉDISTIQUE

(30) Priorität: 18.11.2022 DE 102022130585
(43) Veröffentlichungstag der Anmeldung: 24.09.2025
(73) Patentinhaber: Otto Bock Healthcare Products GmbH, 1110 Wien (AT)
(72) Erfinder: SAUBERER, Wolfgang, 1110 Wien (AT)
(74) Vertreter: Gramm, Lins & Partner Patent- und Rechtsanwälte PartGmbB
(86) Internationale Anmeldenummer: PCT/EP2023/080951
(87) Internationale Veröffentlichungsnummer: WO 2024/104820

(56) Entgegenhaltungen:
- US-A- 3 976 057
- US-A1- 2008 097 269
- US-A1- 2013 165 817
- US-A1- 2022 192 846
- US-B2- 10 746 272

## Beschreibung

Die Erfindung betrifft eine orthopädietechnische Einrichtung mit einem Oberteil und einem verlagerbar daran gelagerten Unterteil, das mit einem Aktuator gekoppelt ist, der das Unterteil relativ zu dem Oberteil verlagert, wobei der Aktuator einen Elektromotor, einen Energiespeicher, eine Steuereinrichtung und einem Spannungsumwandler aufweist. US 2013/165817 A1 stellt den nächsten Stand der Technik dar.

Orthopädietechnische Einrichtungen sind insbesondere Prothesen, Orthesen und Exoskelette. Prothesen ersetzen nicht oder nicht mehr vorhandene Gliedmaßen oder Körperteile soweit möglich hinsichtlich Form und/oder Funktion. Die einfachsten Prothesen haben eine rein kosmetische Funktion oder vollständigen eine Gliedmaße, beispielsweise indem ein distales Fingerglied ersetzt wird. Bei komplexeren prothetischen Versorgungen werden mehrere Prothesenkomponenten miteinander verbunden und aneinander befestigt, insbesondere über Gelenke schwenkbar zueinander miteinander verbunden.

Um die Relativbewegungen der Prothesenkomponenten zueinander zu beeinflussen, wurden Sperreinrichtungen entwickelt, mit denen es möglich ist, ein gestrecktes Prothesenkniegelenk zu verriegeln und zu entriegeln, damit sich der Prothesennutzer hinsetzen kann. Antriebseinrichtungen wurden entwickelt, um über Kabelzüge Bewegungen in der Schulter zu Prothesenhänden übertragen, sodass Gegenstände gegriffen und gehalten werden konnten. Zur Beeinflussung eines Bewegungsverhaltens wurden Dämpfer, insbesondere Hydraulikdämpfer, sowie Energiespeicher, insbesondere in Gestalt von Federn, an den einzelnen Komponenten angeordnet. Ziel dabei ist es unter anderem, eine bestmögliche Funktionalität zu erreichen und gegebenenfalls einen möglichst natürlichen Bewegungsablauf zu ermöglichen.

Zur Unterstützung oder zum Abbremsen von Bewegungen von Prothesenkomponenten wurden in die Prothese Antriebe integriert, sodass aktive Prothesen entstanden sind. Entsprechende Ausgestaltungen sind auch bei passiven und aktiven Orthesen oder Exoskeletten vorhanden, die an vorhandenen Gliedmaßen angeordnet werden. Gelenkübergreifende Orthesen und Exoskelette können ebenfalls mit Federn, Kraftspeichern und/oder motorischen Antrieben ausgestattet sein.

Zur Steuerung sowohl passiver als auch aktiver Prothesen, Orthesen und Exoskelette sind Steuerungseinrichtungen an orthopädietechnischen Einrichtungen angeordnet, die auf der Basis von Sensorwerten oder eingespeicherten Abläufen Steuerungssignale erzeugen, um Antriebe zu aktivieren, zu deaktivieren oder zu modulieren oder um Widerstände zu verändern, beispielsweise durch das Öffnen oder Schließen von Ventilen, das Freigeben oder das Sperren von gespeicherter Energie aus Energiespeichern, das Einschalten, Ausschalten oder Modulieren von Elektromotoren oder dergleichen.

Die US 2013/0 165.817 A1 betrifft einen Kraftmesssensor, der in einer Orthese eingesetzt werden kann und sowohl resistive als auch kapazitive Technologien zur verbesserten Genauigkeit und Zuverlässigkeit einsetzt. Zwei kapazitive Sensorschichten und eine dazwischen angeordnete resistive Schicht verändern ihre Kapazitäten bzw. ihren Widerstand in Abhängigkeit von der auf dem Sensor ausgeübten Kraft.

Die US-A-3 976 057 betrifft eine Orthese mit einem Oberteil und einem Unterteil, die gelenkig miteinander verbunden sind. Das Oberteil und das Unterteil wird jeweils über eine Manschette an dem Oberschenkel bzw. dem Unterschenkel angelegt. Zwischen dem Oberteil und dem Unterteil eine Kolben-Zylinder-Anordnung mit einem Überströmventil vorhanden, um einen Widerstand gegen eine Beugung oder Streckung bereitzustellen. Über ein Ventil kann Luft in den Zylinder gepumpt werden, um eine gesteuerte Beugung oder Streckung zu bewirken.

Die US 2008/0097269 A1 betrifft eine Orthese mit einer Elektro-rheologischen Fluidbremse und gegebenenfalls einem Aktuator, um einen kontrollierbaren Widerstand mit oder ohne den aktiven Antrieb bereitzustellen. Die Steuerung kann manuell oder computergesteuert erfolgen und in jeder Drehrichtung eine Unterstützung und/oder einen Widerstand bieten.

Die US 2022 / 0 192 846 A1 betrifft eine AFO zu Erzeugung eines Drehmomentes um ein Knöchelgelenk mit einem Unterschenkelteil, das über eine Manschette an dem Unterschenkel festlegbar ist. Ein Antrieb ist mit einem Fußteil gekoppelt, das auf der Höhe des natürlichen Knöchelgelenkes gelenkig an dem Unterschenkelteil gelagert ist und eine Plantarflexion und eine Dorsalflexion zulässt. Das Fußteil lässt eine Eversion und eine Inversion zu.

Die US 10 746 272 B2 betrifft eine Bewegungsassistenzvorrichtung mit einer Antriebsquelle, zwei Stützelementen und einem damit verbundenen Kraftübertragungsgestell, das von der Antriebsquelle über eine Kraftübertragungsvorrichtung angetrieben wird. Die Kraftübertragungsvorrichtung umfasst eine eingangsseitige Zahnradanordnung mit einem ersten Krafteingangsanschluss und einer Vielzahl von ersten Kraftausgangsanschlüssen und eine ausgangsseitige Zahnradanordnung mit einer Vielzahl von zweiten Krafteingangsanschlüssen und einem zweiten Kraftausgangsanschluss, wobei die Vielzahl von zweiten Krafteingangsanschlüssen jeweils mit der Vielzahl von ersten Kraftausgangsanschlüssen der eingangsseitigen Zahnradanordnung kraftübertragend verbunden ist. Ein Stoppermechanismus ist dazu ausgelegt, für die Kraftübertragung einen von einer Vielzahl von Kraftübertragungspfaden, die die Vielzahl von ersten Kraftausgangsanschlüssen mit der Vielzahl von zweiten Krafteingangsanschlüssen verbindet, auszuwählen. Der erste Krafteingangsanschluss ist mit der Antriebsquelle verbunden ist, während der zweite Kraftausgangsanschluss mit dem Kraftübertragungsgestell verbunden ist.

Bei aktiven orthopädietechnischen Einrichtungen mit elektromotorischen Antrieben besteht der Energiespeicher in der Regel aus einer Batterie oder einem Akkumulator. Bei einem elektromotorischen Antrieb besteht eine Proportionalität zwischen der benötigten Versorgungsspannung und der Drehzahl des Antriebes, ebenso wie eine Proportionalität zwischen dem Moment des Antriebes und dessen Motorstrom. Die Maximalwerte für Strom und Spannung der Energieversorgung bestimmen somit das maximale Drehmoment ebenso wie die maximale Drehzahl des Antriebes.

Um sowohl hohe Drehmomente als auch hohe Drehzahlen zum Antreiben einer orthopädietechnischen Einrichtung bereitstellen zu können, müssen entsprechend große Energiespeicher bzw. Elektromotoren eingesetzt werden. Da dies das Gewicht der orthopädietechnischen Einrichtung vergrößert, sind der Vergrößerung der Energiespeicher und/oder Motoren Grenzen gesetzt. Ein Elektromotor ist ein elektromechanischer Wandler, der elektrische Energie in mechanische Energie umwandelt. Unter einem Elektromotor werden insbesondere Gleichstrommotoren, Wechselstrommotoren, piezoelektrische Aktuatoren, durch Lorenzkraft wirkende Motoren, Reluktanz-Motoren und thermo-elektrische Aktuatoren verstanden.

Aufgabe der vorliegenden Erfindung ist es, eine orthopädietechnische Einrichtung bereitzustellen, die mit einem vergleichsweise leichten Energiespeicher auskommt bzw. deren Antrieb einen größeren Arbeitsbereich aufweist.

Gelöst wird diese Aufgabe durch eine orthopädietechnische Einrichtung mit den Merkmalen des Hauptanspruches. Vorteilhafte Ausgestaltungen und Weiterbildungen der Erfindung sind in den Unteransprüchen, der Beschreibung sowie den Figuren offenbart.

Die orthopädietechnische Einrichtung mit einem Oberteil und einem verlagerbar daran gelagerten Unterteil, das mit einem Aktuator gekoppelt ist, der das Unterteil relativ zu dem Oberteil verlagert, wobei der Aktuator zumindest einen Elektromotor, einen Energiespeicher und eine Steuerungseinrichtung aufweist, sieht vor, dass zwischen dem Energiespeicher und dem Elektromotor ein erster Umschalter angeordnet ist, der mit der Steuerungseinrichtung gekoppelt ist und die Versorgungsspannung einer Motorsteuerung des Elektromotors zwischen der Ausgangsspannung des Energiespeichers und einem Spannungswandler mit einer gegenüber der Ausgangsstellung veränderten, insbesondere erhöhten Versorgungsspannung umschaltet. Zwischen der Motorsteuerung und dem Energiespeicher ist der Umschalter angeordnet, der in der Steuerungseinrichtung angeordnet ist und die Versorgung der Motorsteuerung entweder direkt durch den Energiespeicher mit dessen Ausgangsspannung oder mit einer erhöhten Spannung bewirkt. Aktive orthopädietechnische Einrichtungen müssen neben einer gewissen Schnelligkeit der Verstellung auch ein ausreichendes Drehmoment aufbringen, um die gewünschten Bewegungen oder Aktionen ausführen zu können oder um eine Bewegungssituation auf die beabsichtigte Art und Weise beeinflussen zu können. Schnelle Bewegungen sind dabei häufig ohne eine große Belastung mit einem geringen Drehmoment auszuführen, während Bewegungen, die ein hohes Drehmoment fordern, in der Regel langsam ausgeführt werden. Mit der Steuerungseinrichtung und dem Umschalter ist es möglich, in einem ersten Schalterzustand die Ausgangsspannung unmittelbar zu dem Elektromotor durchzuleiten, sodass ein hoher Strom mit einer vergleichsweisen geringen Versorgungsspannung, die gleich der Ausgangsspannung des Energiespeichers ist, dem Motor zugeführt wird. Damit ist es möglich, den Elektromotor ein hohes Drehmoment erzeugen zu lassen. Wird von der Steuerungseinrichtung ein entsprechendes Schaltsignal ausgegeben, wird der Spannungswandler eingeschaltet oder aktiviert. Der Spannungswandler verändert, insbesondere erhöht oder verringert die von dem Energiespeicher kommende Ausgangsspannung und führt die erhöhte oder verringerte Spannung als Versorgungsspannung über die Motorsteuerung dem Elektromotor zu. Dadurch wird die Drehzahl oder die mögliche Drehzahl des Elektromotors vergrößert bzw. die maximale Drehzahl des Elektromotors erhöht, einhergehend mit einem verringerten maximalen Drehmoment oder verringert, mit einem entsprechend vergrößerten maximalen Drehmoment. Die direkte Verschaltung der Motorsteuerung mit dem Energiespeicher ermöglicht einen hohen Stromfluss im Motor. Dadurch kann der Elektromotor bei niedrigen Drehzahlen hohe Momente erzeugen. Werden höhere Drehzahlen benötigt, wird auf eine erhöhte Versorgungsspannung durch den Spannungswandler umgeschaltet. Die Steuerungseinrichtung liefert dabei das Schaltsignal zur Einstellung der Versorgungsspannung. Die Steuerungseinrichtung ist in der Lage, den erhöhten Spannungsbedarf bzw. einen Bedarf an einem erhöhten Drehmoment des Motors zu detektieren und eine entsprechende Versorgungsspannung durch Umschaltung des Spannungswandlers bereitzustellen. Dies geschieht beispielsweise durch eine Auswertung von Sensoren, die Bewegungen, Belastungen, Zustände und/oder Positionen oder deren Veränderungen von Prothesen- oder Orthesenkomponenten, der Umgebung und/oder der unversorgten, kontralateralen Seite des Patienten erfassen. Die Auswertung der Sensoren kann auch dergestalt erfolgen, dass aus den bisherigen Signalverläufen auf den weiteren, zukünftigen Verlauf geschlossen wird.

**In** einer Ausgestaltung ist die Ausgangsspannung des Spannungswandlers dynamisch einstellbar, um ein weiches Umschalten zwischen den jeweils bereitgestellten bzw. benötigten Versorgungsspannungen zu ermöglichen. Um den erhöhten Spannungsbedarf in einzelnen Phasen eines Schrittes abdecken zu können, erfolgt eine bedarfsgerechte Anpassung der Ausgangsspannung des Spannungswandlers. Durch die bedarfsgerechte Anpassung werden die Verluste innerhalb des Spannungswandlers, die durch das Hochsetzen der Spannung auftreten, möglichst gering gehalten. Die dynamische Einstellbarkeit ermöglicht ein weiches Umschalten der jeweils bereitgestellten Versorgungsspannung.

Der Spannungswandler ist in einer Ausgestaltung der Gestalt ausgebildet, dass er sowohl aufwärts als auch abwärts wandeln kann, also eine erhöhte oder eine verringerte Spannung als Versorgungsspannung bereitstellen kann. Alternativ ist ein Aufwärtswandler und ein Abwärtswandler in einer Ausgestaltung in dem Spannungswandler vorgesehen, um die jeweils gewünschte Versorgungsspannung bereitstellen zu können.

**In** einer Ausgestaltung ist der Spannungswandler bidirektional ausgeführt und weist einen Aufwärtswandler und einen Abwärtswandler auf oder weist nur einen Wandler auf, der sowohl aufwärts als auch abwärts wandeln kann. Somit können im Generatorbetrieb des Motors, wenn Energie in die Batterie bzw. in den Energiespeicher zurück gespeist wird, die rückgeführte Spannung eingestellt werden. Einerseits kann im Vorwärtsbetrieb die Ausgangsspannung des Energiespeichers auf ein höheres Niveau zur Versorgung der Motorsteuerung angehoben werden, andererseits ermöglicht der Abwärtswandler den Rückwärtsbetrieb. Dabei arbeitet der Elektromotor bei einem erhöhten Versorgungsspannungsniveau im Generatorbetrieb. Die Abwärtswandlung ermöglicht es, die im Motor entstehende elektrische Energie kontrolliert zurück in den Energiespeicher zu laden. Der Generatorbetrieb ermöglicht einen Rückfluss von elektrischer Energie in den Energiespeicher, die erzeugt wird, während der Elektromotor bei einem erhöhten Spannungsniveau der Versorgungsspannung im Generatorbetrieb arbeitet. Insbesondere bei einem dynamisch einstellbaren Spannungswandler ist es damit möglich, die Verluste im Aufwärtswandler bzw. Abwärtswandler zu reduzieren, wenn die Spannung von dem Motor abwärts oder aufwärts gewandelt wird.

Eine Weiterbildung sieht vor, dass ein zweiter Umschalter zum Umschalten zwischen dem Aufwärtswandler und dem Abwärtswandler mit der Steuerungseinrichtung gekoppelt ist. Die Notwendigkeit für einen zweiten Umschalter ist insbesondere dann gegeben, wenn der Ausgang des Spannungswandlers nicht deaktiviert werden kann. Bei einer solchen Ausgestaltung des Spannungswandlers muss der zweite Umschalter immer dann sperren, wenn der erste Umschalter durchlässt bzw. muss der zweite Umschalter immer durchlassen, wenn der erste Umschalter gesperrt ist.

In der Ausgangsstellung des ersten Umschalters liegt die Ausgangsspannung des Energiespeichers als Versorgungsspannung der Motorsteuerung des Elektromotors an, sodass zunächst mit einer geringen Maximaldrehzahl und einem maximalen Drehmoment gearbeitet wird. Erst bei Vorliegen eines entsprechenden Steuerungssignals, beispielsweise auf Grundlage von Sensoren, wird eine Umschaltung auf eine Erhöhung der Versorgungsspannung durchgeführt.

In einer Ausgestaltung ist der Umschalter oder sind die Umschalter als Teil des Spannungswandlers ausgebildet. Die Umschaltung kann somit teilweise oder vollständig in dem Spannungswandler integriert sein.

In einer Ausgestaltung ist die Versorgungsleitung der Motorsteuerung elektrisch von dem Spannungswandler und von dem Energiespeicher entkoppelt. Die Umschaltung und der Spannungswandler können vollständig oder teilweise in der Motorsteuerung integriert sein. In einer Ausgestaltung bewirkt der Umschalter die Umschaltung der Schaltungskonfiguration des Energiespeichers.

In einer Weiterbildung besteht der Spannungswandler aus zumindest einem Halbleiterschalter, wobei zumindest einer dieser Halbleiterschalter als Teil der Umschaltvorrichtung ausgebildet sein kann.

Eine Weiterbildung sieht vor, dass die Steuerungseinrichtung dergestalt ausgebildet ist, dass eine Erhöhung der Versorgungsspannung der Motorsteuerung erst bei Überschreiten einer Drehzahlschwelle des Elektromotors vorgenommen wird. Die Versorgungsspannung der Motorsteuerung kann von der Motordrehzahl, dem Motormoment, dem Motorstrom und/oder deren zeitlichen Verläufen abhängen. Eine Motordrehzahl muss zur Ermittlung, ob eine Drehzahlschwelle erreicht und überschritten ist, nicht gemessen werden, sondern es kann auch eine Änderungsrate eines damit gekoppelten Freiheitsgrades gemessen werden und damit auf das Überschreiten einer Drehzahl oder einer Drehzahlschwelle eines Motors geschlossen werden.

Nachfolgend werden Ausführungsbeispiele der Erfindung anhand der beigefügten Figuren näher erläutert. Es zeigen:
Figur 1 - eine schematische Darstellungen einer Prothese;
Figur 2 - eine schematische Darstellung einer Orthese;
Figur 3 - ein Blockschaltbild einer Spannungsversorgung;
Figur 4 - eine Plotdarstellung von Gangdaten;
Figur 5 - eine exemplarische Blockdarstellung mit unterschiedlichen Arbeitsbereichen;
Figur 6 - ein alternatives Schaltbild;
Figur 7 - der Energie- und Signalfluss in dem Schaltbild gemäß Figur 6;
Figur 8 - eine Variante der Figur 6.

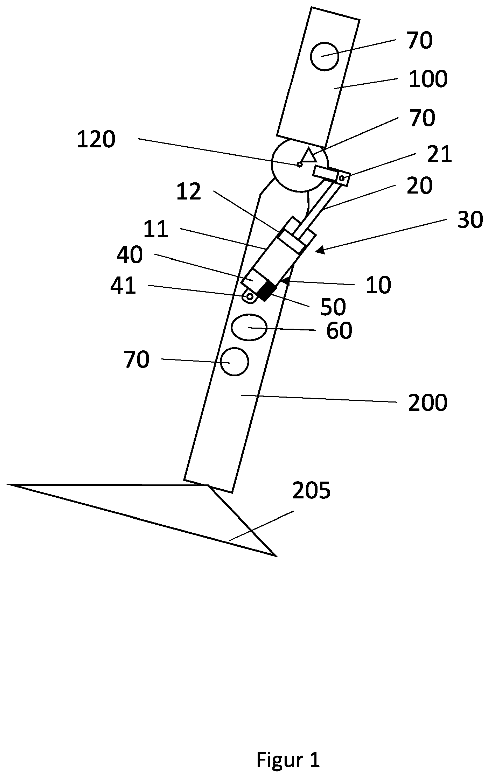

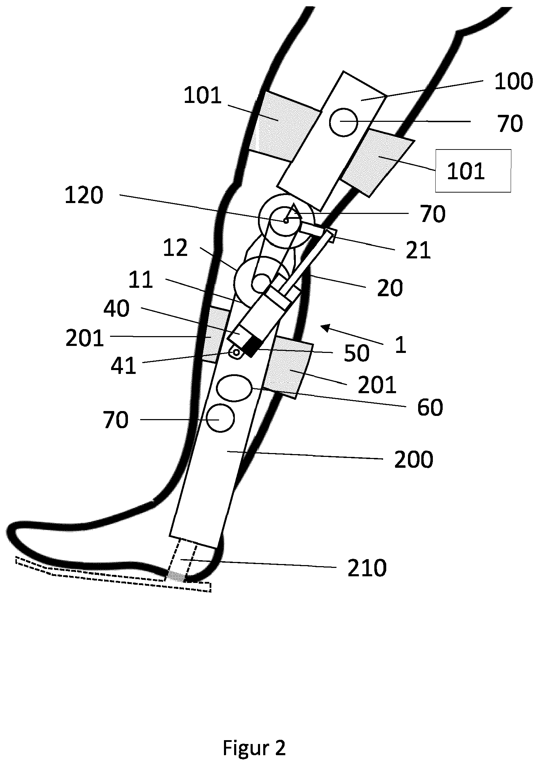

**In** der Figur 1 ist in einer schematischen Darstellung ein künstliches Kniegelenk als Teil einer Prothese und in der Figur 2 als Teil einer Orthese als orthopädietechnische Einrichtung dargestellt. Das künstliche Kniegelenk weist ein Oberteil 100 und ein Unterteil 200 auf, die um eine Schwenkachse 120 schwenkbar aneinander gelagert sind. An dem Unterteil 200 ist bei einer Ausgestaltung als Prothese ein Prothesenfuß 205 an dem distalen Ende angeordnet, bei der Ausgestaltung des künstlichen Kniegelenkes als ein Orthesenkniegelenk, wie in Figur 2 gezeigt, ist das Unterteil 200 als Unterschenkelschiene ausgebildet, an der kein Fußteil angeordnet ist, aber an dem ein optionales Fußteil 210 angeordnet werden kann, das in der unterbrochenen Linie dargestellt ist. Für den Fall einer KAFO ist an dem Unterteil 200 ein Fußteil 210 angeordnet, auf dem ein Fuß aufgesetzt werden kann. Dieses kann aber zur Realisierung einer reinen Knieorthese auch weggelassen werden. In der Ausgestaltung als Prothesenbein gemäß Figur 1 ist an dem Oberteil 100 ein Prothesenschaft oder eine andere Einrichtung zur Aufnahme eines Oberschenkelstumpfes oder zur Festlegung an einer Person angeordnet oder ausgebildet. Bei der Ausgestaltung gemäß Figur 2 erfolgt eine Festlegung der Orthese an einem Bein über Befestigungsmittel 101, 201, die beispielsweise als Gurte, Schalen oder dergleichen ausgebildet sind, um die Orthese abnehmbar an dem Bein festzulegen. Ein weiterer Unterschied zwischen der Ausgestaltung gemäß der Figur 1 und der Ausgestaltung gemäß der Figur 2 ist, dass gemäß der Figur 2 ein alternativer Antrieb vorgesehen ist, bei dem ein Elektromotor 40, gegebenenfalls über ein Getriebe, mit einer Riemenscheibe gekoppelt ist. Über einen Keilriemen oder Zahnriemen kann dann je nach Drehrichtung des Motors eine Flexion oder Extension des Kniegelenkes bewirkt bzw. unterstützt werden. Die Ausführung mit dem Antrieb mit einem Elektromotor 40 über eine mechanische Kraftübertragungseinrichtung sowie eine parallele Dämpfung über einen Hydraulikdämpfer kann auch bei einem Prothesenkniegelenk oder einer anderen prothetischen Einrichtung angewendet werden.

Zwischen dem Oberteil 100 und dem Unterteil 200 ist ein Aktuator 30 als ein linear wirkender Hydraulikmotor angeordnet. In dem dargestellten Ausführungsbeispiel ist der hydraulische Aktuator 30 mit einer Hydraulikkammer oder einem Zylinder 11 ausgebildet, der in einem Gehäuse oder Grundkörper 10 angeordnet oder ausgebildet ist. In dem Zylinder 11 ist ein Kolben 12 verschieblich gelagert. Der Kolben 12 ist entlang der Längserstreckung des Zylinders 11 verlagerbar und an einer Kolbenstange 20 befestigt, die aus dem Gehäuse oder Grundkörper 10 hinausragt. Der Kolben 12 unterteilt den Zylinder 11 in Kammern, die über eine Hydraulikleitung in strömungstechnischer Verbindung miteinander stehen. Der Grundkörper 10 oder das Gehäuse kann verschwenkbar an dem Unterteil 200 gelagert sein, um eine Verkantung des Kolbens 12 bei einer Verschwenkbewegung des Oberteils 100 relativ zu dem Unterteil 200 zu verhindern. Das dem Kolben 12 abgewandte Ende der Kolbenstange 20 ist an dem Oberteil 100, in dem dargestellten Ausführungsbeispiel an einem Ausleger zur Vergrößerung des Abstandes zu der Schwenkachse 120, befestigt. Bei einer Flexion wird der Kolben 12 nach unten gedrückt, sodass sich das Volumen einer Flexionskammer verkleinert, korrespondierend dazu vergrößert sich das Volumen einer Extensionskammer, verringert um das Volumen der einfahrenden Kolbenstange 20. **In** dem Gehäuse 10 ist zur Erzeugung eines Druckes innerhalb einer der Kammern ein Elektromotor 40 angeordnet, der eine nicht dargestellte Pumpe eintreibt, um das Hydraulikfluid innerhalb einer der beiden Kammern mit einem Druck zu beaufschlagen und dadurch den Kolben 12 innerhalb des Zylinders in die eine oder andere Richtung zu bewegen. Dadurch wird eine Flexionsbewegung oder eine Extensionsbewegung der orthopädietechnischen Einrichtung in Gestalt des Prothesenbeines bewirkt.

**In** dem dargestellten Ausführungsbeispiel ist der Aktuator 30 über eine Befestigungseinrichtung 41 an dem Unterteil 200 festgelegt. Sowohl an dem Oberteil 100 als auch an dem Unterteil 200 ist ein Sensor 70 zur Erfassung der Raumorientierung des Unterteils 200 bzw. des Oberteils 100 angeordnet. Über diesen Sensor 70, der beispielsweise als IMU (inertial measurement unit) ausgebildet sein kann, wird während der Benutzung des künstlichen Kniegelenkes der Raumwinkel oder der Absolutwinkel zu einer festen Raumorientierung, beispielsweise der Gravitationsrichtung, ermittelt. Statt einer IMU kann der Sensor 70 auch andere Zustandsdaten erfassen, insbesondere Zustandsdaten, die das künstliche Kniegelenk betreffen. Als Zustandsdaten werden insbesondere Positionen, Winkelstellungen, Geschwindigkeiten, Beschleunigungen, Kräfte sowie deren Verläufe oder Änderungen erfasst. Der ermittelte Raumwinkel des Oberteils 100 und/oder des Unterteils 200 oder eine andere Zustandsgröße wird mit einem Schwellwinkel verglichen. Bei Erreichen oder Überschreiten eines Schwellwertes, der in einer Steuerung für den jeweiligen Sensorwert oder einer daraus abgeleiteten Größe abgelegt ist, wird ein Aktuator aktiviert oder deaktiviert, um den Strömungswiderstand in dem Aktuator 30 zu verändern.

Der Aktuator 30 in einem künstlichen Kniegelenk dient dazu, eine Flexionsbewegung und eine Extensionsbewegung zu moderieren, um einen angemessenen oder gewünschten Bewegungsablauf zu erzeugen oder zu unterstützen. Eine Extensionsbewegung wird ggf. unterstützt und vorteilhafterweise kurz vor Erreichen einer maximalen Streckung abgebremst, um ein hartes Anschlagen zu vermeiden. Eine Flexionsbewegung wird in der Standphase und in der Schwungphase abgebremst oder unterbunden, um eine Begrenzung der Einbeugung zu gewährleisten. Um den Elektromotor 40 antreiben zu können, ist ebenfalls ein Energiespeicher 50 insbesondere in Gestalt eines Akkumulators in dem Aktuator 30 angeordnet. Der Energiespeicher 50 kann auch an einer anderen Stelle der orthopädietechnischen Einrichtung angeordnet sein, wo mehr Raum zur Verfügung steht oder wo dies aufgrund der Gewichtsverteilung vorteilhaft erscheint.

Alternativ zu der Ausgestaltung des Aktuators 30 als Hydraulikmotor mit einem Elektromotor 40 und einer Pumpe kann der Aktuator 30 auch eine direkte mechanische Kopplung des Elektromotors 40 mit dem Oberteil 100 und dem Unterteil 200 über eine Kraftübertragungseinrichtung aufweisen, beispielsweise über einen Spindelantrieb, so dass statt einer Kolbenstange 20 eine Spindel durch das Verdrehen einer Spindelmutter, die durch den Elektromotor 40 angetrieben wird, aus dem Gehäuse 11 eingefahren bzw. ausgefahren wird. Der Aktuator 30 ist in einer Ausführungsform über eine Getriebeeinrichtung mit dem Oberteil 100 und dem Unterteil 200 gekoppelt, beispielsweise über ein Planetengetriebe, um eine Verlagerung des Oberteils 100 relativ zu dem Unterteil 200 zu bewirken. Alternativ zu einer Ausgestaltung der orthopädietechnischen Einrichtung als eine Orthese oder Prothese oder ein Exoskelett der unteren Extremität kann diese auch als eine Orthese oder Prothese oder ein Exoskelett der oberen Extremität ausgebildet sein. Auch andere orthopädietechnische Einrichtungen mit zwei zueinander verlagerbaren Komponenten, deren Relativbewegungen zueinander über einen Elektromotor beeinflusst werden, machen von der Erfindung Gebrauch.

Weiterhin sind an der orthopädietechnischen Einrichtung eine Steuerungseinrichtung 60 sowie zumindest eine Winkelerfassungseinrichtung 70 als Sensor angeordnet. Die Winkelerfassungseinrichtung 70 erfasst den Winkel zwischen dem Oberteil 100 und dem Unterteil 200 und ist beispielsweise als direkter Winkelsensor ausgebildet, der unmittelbar den Winkel detektiert. Alternativ kann der Winkel zwischen dem Oberteil 100 und dem Unterteil 200 über eine Auswertung der Sensordaten der Raumlagesensoren 70 ermittelt werden. Beide Verfahren können auch gleichzeitig oder einander ergänzend eingesetzt werden. Alle an der orthopädietechnischen Einrichtung angeordneten Sensoren sind mit einer Steuerungseinrichtung 60 gekoppelt und deren Sensorwerte dienen als Grundlage zur Steuerung des Aktuators 30. Auf der Grundlage der Sensordaten, insbesondere der Raumlagen und/oder Winkelpositionen ebenso wie Positionsdaten und Daten zur Belastung, Orientierung, Beschleunigung und/oder Deformation weiterer Komponenten wird der Aktuator 30 angesteuert, beispielsweise um den Elektromotor 40 zu aktivieren, deaktivieren oder zu moderieren, beispielsweise um einen Verschwenkungswiderstand zu verringern oder zu vergrößern, einen Endanschlag zu begrenzen und/oder um eine Relativbewegung zwischen dem Oberteil 100 und dem Unterteil 200 zu erzeugen oder zu unterstützen.

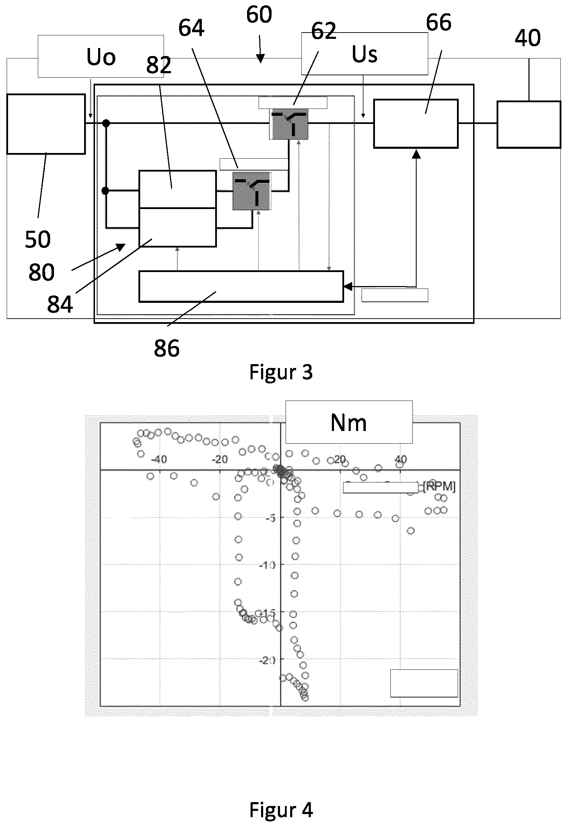

In der Figur 3 ist ein Schaltbild der Spannungsversorgung des Elektromotors 40 von dem Energiespeicher 50 dargestellt. Zwischen dem Elektromotor 40 und dem Energiespeicher 50 ist die Steuerungseinrichtung 60 angeordnet, die mehrere Komponenten aufweist, um den Elektromotor 40 mit der jeweils notwendigen oder gewünschten Versorgungsspannung Us zu versorgen. Von dem Energiespeicher 50 wird die Ausgangsspannung Uo abgegeben und der Motorsteuerung 66 zunächst unverändert zugeführt. Innerhalb der Steuerungseinrichtung 60 befindet sich ein erster Umschalter 62 und eine unmittelbare Motorsteuerung 66, die auf Grundlage von Daten, insbesondere Sensordaten, ermittelt und bestimmt, welche Versorgungsspannung Us dem Elektromotor 40 zugeführt wird oder werden muss, um ein optimales Ergebnis zu erreichen. In einer Ausgangstellung befindet sich der erste Umschalter 62 in einer durchleitenden Stellung, bei der die Ausgangsspannung Uo von dem Energiespeicher 50 direkt als Versorgungsspannung Us der Motorsteuerung 66 zugeführt wird. Somit können, bei der Ausgangsspannung Uo als Versorgungsspannung Us, die maximalen Motorströme bei niedriger Motorspannung dem Elektromotor 40 über die Motorsteuerung 66 zugeführt werden.

Wird über die Motorsteuerung 66 oder eine andere Auswerteinrichtung mit einem Computer detektiert, dass eine erhöhte Drehzahl von dem Elektromotor 40 notwendig ist, beispielsweise um in einem entlasteten Zustand das Unterteil 200 relativ zu dem Oberteil 100 schnell zu verschwenken, gibt die Motorsteuerung 66 ein entsprechendes Steuersignal an einen Spannungsregler 86. Der Spannungsregler 86 kennt die Schalterstellung des ersten Umschalters 62 und gibt einen entsprechenden Befehl an den ersten Umschalter 62 und einen ersten Spannungswandler 82, der als Aufwärtswandler ausgebildet ist. Der Aufwärtswandler 82 transformiert die Ausgangsspannung Uo des Energiespeichers 50 auf ein höheres Niveau und gibt diese Zwischenkreisspannung als Versorgungsspannung Us dann über die Motorsteuerung 66 an den Elektromotor 40 weiter. Das Steuersignal der Motorsteuerung 66 liefert somit den Befehl zur Einstellung der jeweils benötigten Versorgungsspannung Us. Die Umschaltung auf die Versorgungsspannung Us mit einer höheren Spannung im Vergleich zur Ausgangsspannung Uo erfolgt mit dem ersten Umschalter 62, der nun die Ausgangsspannung des Aufwärtswandlers 82 als Us bereitstellt. Der Umschaltvorgang wird vorteilhafterweise durch einen dynamisch einstellbaren Aufwärtswandler 82 begünstigt. Damit ist ein weiches Umschalten zwischen den verschiedenen Spannungsquellenmöglich. Darüber hinaus werden Verluste innerhalb des Aufwärtswandlers 82 zu verringert. Verluste werden insbesondere dadurch verkleinert dass die Ausgangsspannung Uo dem erhöhten Spannungsbedarf folgen kann. Das Ausgangsspannungsniveau wird nur so weit wie benötigt angehoben. Bei einer erhöhten Versorgungsspannung Us im Vergleich zu der Ausgangsspannung Uo sind die maximalen Motorströme verringert, sodass der Elektromotor 40 zwar eine höhere Drehzahl erreichen kann, jedoch nur ein geringeres Drehmoment bereitstellt.

In der dargestellten Ausführungsform ist der Spannungswandler bidirektional ausgeführt und hat neben dem Aufwärtswandler 82 einen Abwärtswandler 84, mit dem es möglich ist, erhöhte Spannungen abzubauen bzw. Energie aus der Bewegung, die über den Motor 40 aufgenommen wird, zu entnehmen und dem Energiespeicher 50 wieder zurückzuführen. Dazu ist der zweite Umschalter 64 dem Spannungsregler 86 zugeordnet, sodass bei entsprechenden Belastungszuständen, Bewegungszuständen und Energiezuständen die Steuerungseinrichtung 60 über die Motorsteuerung 66 und den Spannungsregler 86 die beiden Umschalter 62, 64 ansteuert und je nach Bedarf die Ausgangsspannung Uo erhöht bzw. wieder auf das Ausgangsniveau zurückgesetzt bzw. dem Energiespeicher 50 elektrische Energie zum Wiederaufladen von dem Motor 40 im Generatorbetrieb zurückführt.

Figur 4 stellt einen exemplarischen Verlauf von motorisch generiertem Drehmoment in der Knieachse über der resultierenden Drehzahl während eines Schrittes dar. Die einzelnen Kreise repräsentieren die gemessenen Punkte. Für jeden Schritt ist das jeweilige Ausgangsdrehmoment des Elektromotors in Nm über die Drehzahl pro Minute in rpm aufgetragen. Es ergibt sich eine Korrelation aus einem vergleichsweisen großen Drehmoment von bis zu minus 25 Nm bei der geringen Drehzahl zwischen 10 und minus 15 rpm und eine hohe Verstellgeschwindigkeit von minus 50 rpm bis plus 60 rpm bei einem Ausgangsdrehmoment von nicht mehr als plus minus 5 Newtonmeter. Charakteristisch für die Anwendung eines aktiven Antriebes einer Knieprothese ist die große Spreizung zwischen den Bereichen mit einem hohen Drehmoment und niedrigen Drehzahlen sowie den Bereichen mit einem niedrigen Drehmoment und hohen Drehzahlen. Bei einem elektrischen Antrieb mit einem Elektromotor besteht eine Proportionalität zwischen der benötigten Versorgungsspannung Us und der Drehzahl des Antriebes ebenso wie eine Proportionalität zwischen dem Drehmoment des Antriebes und dessen Motorstrom. Die Maximalwerte für den Strom und die Versorgungsspannung Us bestimmen somit einerseits das maximale Drehmoment und andererseits die maximale Drehzahl des Antriebes. Ein Energiespeicher ist üblicherweise aus mehreren Zellen eines Akkumulatorsystems aufgebaut. Bei einer gegebenen Anzahl von Zellen kann man das Gesamtsystem entweder für hohe Ströme durch eine Parallelschaltung mehrerer Zellen optimieren oder man optimiert für hohe Spannungen durch eine serielle Verschaltung der Zellen. Will man beide Bereiche, also hohe Drehmomente und hohe Drehzahlen, gleichermaßen erreichen, müssen weitere Zellen hinzugefügt werden. Dadurch vergrößern sich das Volumen und damit auch das Gewicht des Energiespeichers.

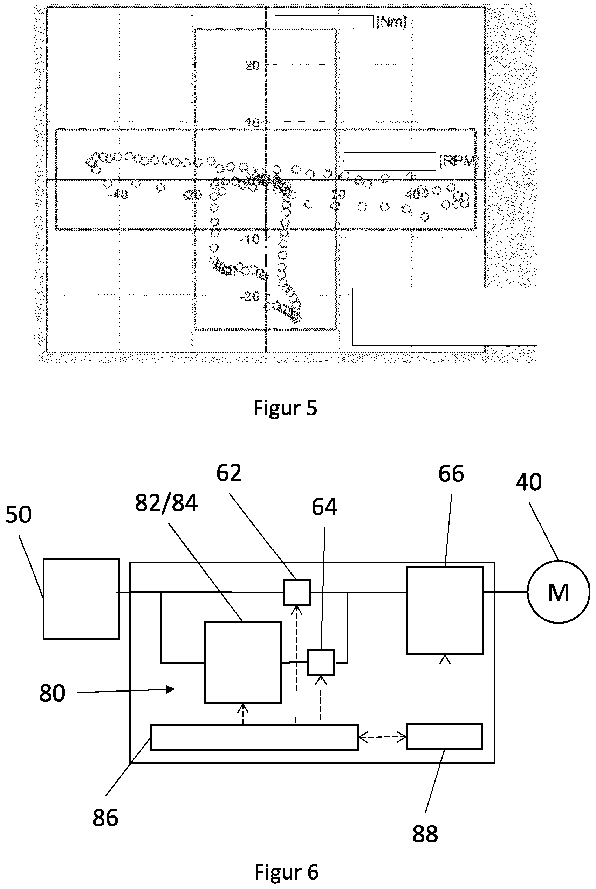

In der Figur 5 ist ein exemplarischer Plot von Gangdaten einer aktiven Knieprothese mit einer Energieversorgung gemäß einer Schaltung gemäß der Figur 3 dargestellt. Das motorisch generierte Drehmoment in der Knieachse über der resultierenden Drehzahl während eines Schrittes ist dargestellt. Die einzelnen Kreise repräsentieren die gemessenen Punkte. Eingezeichnet sind die beiden Arbeitsbereiche, zwischen denen durch die Umschalter 62, 64 umgeschaltet wird. Das vertikale Rechteck symbolisiert den Bereich mit einer Versorgungsspannung Us, die der Ausgangsspannung Uo entspricht, also ein vergleichsweises hohes Drehmoment bei einer vergleichsweise geringen Drehzahl ermöglicht. Das horizontal orientierte Rechteck ist der Bereich, in dem der Elektromotor 40 bei einer gegenüber der Ausgangsspannung Uo erhöhten Versorgungsspannung Us arbeitet, sodass eine höhere maximale Drehzahl möglich ist, allerdings bei einem verringerten maximalen Drehmoment. Das vertikal orientierte Rechteck zeigt den Betriebsbereich an, bei dem ein maximaler Ausgangsstrom bei der Ausgangsspannung Uo dem Elektromotor 40 zugeführt werden kann, wodurch der Bereich der hohen Drehmomente abgedeckt wird. Der horizontal orientierte Arbeitsbereich stellt den durch den Spannungsregler 86 und den Aufwärtswandler 82 erhöhten Spannungsbereich bei einem reduzierten Maximalstrom dar. Hohe Drehzahlen bei vergleichsweise kleinen Momenten können abgedeckt werden. Der Überlappungsbereich beider Bereiche wird vorteilhafterweise durch den vertikalen Arbeitsbereich bei direkt durchgeleiteter Ausgangsspannung Uo ohne Zwischenschaltung eines Wandlers 82 abgedeckt.

Die Spannungswandler 82, 84 sind dynamisch einstellbar, der Abwärtswandler 84 wandelt, während Phasen mit erhöhter Motorspannung, im Generatorbetrieb des Elektromotors 40 aus der Bewegung erzeugte elektrische Energie in die entsprechende Spannung zur Aufladung des Energiespeichers 50 um. In Phasen mit Motorspannungen Us kleiner als der Ausgangsspannung Uo und deaktiviertem Spannungswandler 84, wenn eine direkte Verbindung zu Energiespeicher 50 Vorhanden, kann der Elektromotor 40 den Energiespeicher 50 über die Motorsteuerung 66 aufladen. Die Hochsetzung erlaubt auch, dass das Verhalten der orthopädietechnischen Einrichtung hinsichtlich der erreichbaren Drehzahlen auch bei sinkender Spannung bei einem sich entleerenden Energiespeicher 50 gleich bleibt.

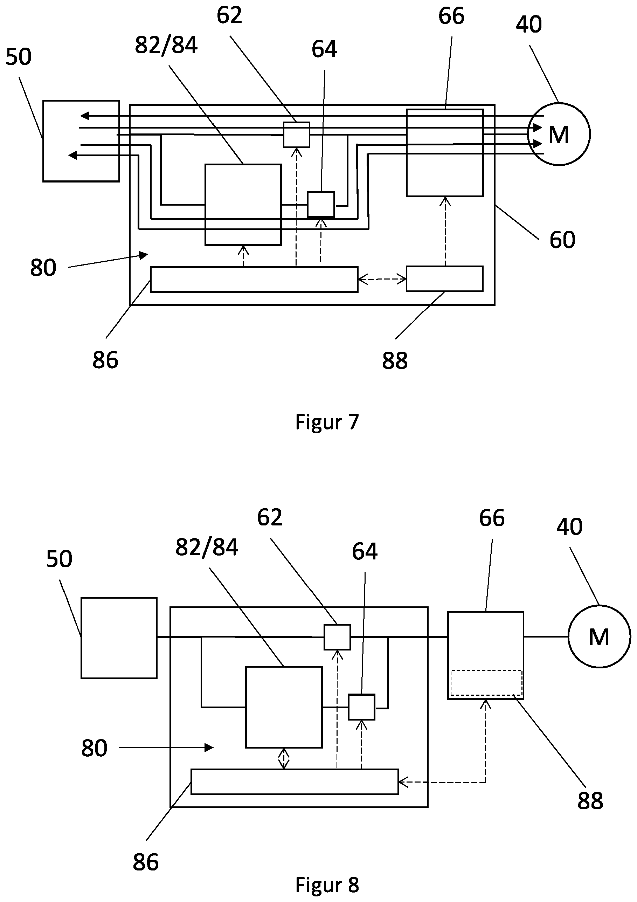

In der Figur 6 ist ein Schaltbild einer Motorsteuerung als Alternative zu der Motorsteuerung gemäß Figur 3 dargestellt, bei der die gleichen Bezugszeichen die gleichen Bauteile bezeichnen. In der Figur 7 sind die jeweiligen Energieflüsse durch durchgehende Pfeile dargestellt. Von dem Energiespeicher 50 wird Energie dem Motor 40 zugeführt bzw. im Generatorbetrieb des Motors 40 wird Energie dem Energiespeicher 50 zugeführt. Auch in dieser Ausgestaltung weist die Steuereinrichtung 60 einen ersten Umschalter 62 und einen zweiten Umschalter 64 auf, wobei der zweite Umschalter 64 optional ist. Die Motorsteuerung 66 wird über einen Motorregler 88 geregelt bzw. gesteuert, der wiederum mit dem Spannungsregler 86 verbunden ist und Steuersignale austauscht. Der Austausch der Steuersignale wird durch die gestrichelten Pfeile dargestellt. Der Motorregler 88 beeinflusst auch die Motorsteuerung 66.

Der oberste Pfeil gemäß Figur 7 zeigt den Energiefluss im Generatorbetrieb oder im Umkehrbetrieb, bei dem der Motor 40 angetrieben wird. Die in dem Motor 40 erzeugte elektrische Energie, beispielsweise bei einer Abbremsung eines prothetischen oder orthetischen Gelenkes, wird zurück zu dem Energiespeicher 50 geleitet. Sofern ein hohe Strom und eine niedrige Spannung vorhanden sind, erfolgt die Übertragung ohne Zwischenschaltung eines Spannungswandlers. Umgekehrt wird im herkömmlichen Betrieb, der durch den zweiten Pfeil von oben angedeutet ist, die Energie von dem Energiespeicher 50 direkt ohne Veränderung, gegebenenfalls mit einer Abwärtswandlung, dem Motor 40 zugeführt, sodass bei einem hohen Strom eine vergleichsweise niedrige Versorgungsspannung an dem Motor 40 anliegt.

In dem dritten Pfeil von oben ist der Energiefluss eines Antriebs gezeigt, bei dem eine höhere oder erhöhte Versorgungsspannung oder Motorspannung für den Motor 40 benötigt wird. Von dem Energiespeicher 50 wird die elektrische Energie durch die Steuereinrichtung 60 zu dem entsprechenden Spannungswandler 84 geleitet, um eine erhöhte Motorspannung im Vorwärtsbetrieb zu erzeugen. Umgekehrt, im Rückwärtsbetrieb bei einer erhöhten Motorspannung, was durch den untersten Pfeil dargestellt ist, wird die erzeugte elektrische Energie von dem Motor 40 im Generatorbetrieb durch den Abwärtswandler 82 geleitet und dann an den Energiespeicher 50 übertragen.

In der Figur 8 ist eine Variante der Figur 6 dargestellt, bei der der Motorregler 88 als integraler Teil der Motorsteuerung 66 ausgebildet ist.

Grundsätzlich kann die Energieversorgung auch komplett von der Motorsteuerung 66 oder von dem Motor 40 getrennt werden.

## Patentansprüche

1. Orthopädietechnische Einrichtung mit einem Oberteil (100) und einem verlagerbar daran gelagerten Unterteil (200), das mit einem Aktuator (30) gekoppelt ist, der das Unterteil (200) relativ zu dem Oberteil (100) verlagert, wobei der Aktuator (30) einen Elektromotor (40), einen Energiespeicher (50), eine Steuerungseinrichtung (60) und einem Spannungsumwandler (80) aufweist, **dadurch gekennzeichnet, dass**
zwischen dem Energiespeicher (50) und dem Elektromotor (40) ein erster Umschalter (62) angeordnet ist, der mit der Steuerungseinrichtung (60) gekoppelt ist und die Versorgungsspannung (Us) einer Motorsteuerung (66) des Elektromotors (40) zwischen der Ausgangsspannung (Uo) des Energiespeichers (50) und dem Spannungswandler (80) mit einer gegenüber der Ausgangsspannung (Uo) veränderten Versorgungsspannung (Us) umschaltet.

2. Orthopädietechnische Einrichtung nach Anspruch 1, **dadurch gekennzeichnet, dass** die Ausgangsspannung des Spannungswandlers (80) dynamisch einstellbar ist.

3. Orthopädietechnische Einrichtung nach Anspruch 1 oder 2, **dadurch gekennzeichnet, dass** der Spannungswandler (80) sowohl aufwärts als auch abwärts wandeln kann oder einen Aufwärtswandler (82) und einen Abwärtswandler (84) aufweist.

4. Orthopädietechnische Einrichtung nach Anspruch 3, **dadurch gekennzeichnet, dass** ein zweiter Umschalter (64) zum Umschalten zwischen dem Aufwärtswandler (82) und dem Abwärtswandler (84) mit der Steuerungseinrichtung (60) gekoppelt ist.

5. Orthopädietechnische Einrichtung nach einem der voranstehenden Ansprüche, **dadurch gekennzeichnet, dass** in der Ausgangsstellung des ersten Umschalters (62) die Ausgangsspannung (Uo) des Energiespeichers (50) als Versorgungsspannung (Us) der Motorsteuerung (66) des Elektromotors (40) anliegt.

6. Orthopädietechnische Einrichtung nach einem der voranstehenden Ansprüche, **dadurch gekennzeichnet, dass** der Umschalter (62, 64) als Teil des Spannungswandlers (80) ausgebildet ist.

7. Orthopädietechnische Einrichtung nach einem der voranstehenden Ansprüche, **dadurch gekennzeichnet, dass** der Spannungswandler (80) aus zumindest einem Halbleiterschalter besteht und zumindest einer dieser Halbleiterschalter als Umschalter (62, 64) ausgebildet ist.

8. Orthopädietechnische Einrichtung nach einem der voranstehenden Ansprüche, **dadurch gekennzeichnet, dass** die Steuerungseinrichtung (60) dergestalt ausgebildet ist, dass eine Erhöhung der Versorgungsspannung (Us) der Motorsteuerung (66) erst bei Überschreiten einer Drehzahlschwelle des Elektromotors (40) vorgenommen wird.

9. Orthopädietechnische Einrichtung nach einem der voranstehenden Ansprüche, **dadurch gekennzeichnet, dass** die Versorgungsspannung (Us) der Motorsteuerung (66) oder des Motors (40) von der Motordrehzahl, dem Motormoment, dem Motorstrom und/oder deren zeitlichen Verläufen abhängt.

## Claims

1. An orthopedic device having an upper part (100) and a lower part (200), which is mounted displaceably on the latter and is coupled to an actuator (30) that displaces the lower part (200) relative to the upper part (100), the actuator (30) comprising an electric motor (40), an energy store (50), a control device (60) and a voltage converter (80), **characterized in that** a first selector switch (62) is arranged between the energy store (50) and the electric motor (40) and is coupled to the control device (60), and switches the supply voltage (Us) of a motor controller (66) of the electric motor (40) between the output voltage (Uo) of the energy store (50) and the voltage converter (80) with a supply voltage (Us) that is modified in relation to the output voltage (Uo).

2. The orthopedic device as claimed in claim 1, **characterized in that** the output voltage of the voltage converter (80) is dynamically adjustable.

3. The orthopedic device as claimed in claim 1 or 2, **characterized in that** the voltage converter (80) can convert both up and down or comprises a step-up converter (82) and a step-down converter (84).

4. The orthopedic device as claimed in claim 3, **characterized in that** a second selector switch (64) for switching between the step-up converter (82) and the step-down converter (84) is coupled to the control device (60).

5. The orthopedic device as claimed in one of the preceding claims, **characterized in that**, in the initial setting of the first selector switch (62), the output voltage (Uo) of the energy store (50) is applied as a supply voltage (Us) of the motor controller (66) of the electric motor (40).

6. The orthopedic device as claimed in one of the preceding claims, **characterized in that** the selector switch (62, 64) is configured as part of the voltage converter (80).

7. The orthopedic device as claimed in one of the preceding claims, **characterized in that** the voltage converter (80) consists of at least one semiconductor switch and at least one of the semiconductor switches is configured as a selector switch (62, 64).

8. The orthopedic device as claimed in one of the preceding claims, **characterized in that** the control device (60) is configured in such a way that an increase of the supply voltage (Us) of the motor controller (66) is performed only when a rotational speed threshold of the electric motor (40) is exceeded.

9. The orthopedic device as claimed in one of the preceding claims, **characterized in that** the supply voltage (Us) of the motor controller (66) or of the motor (40) depends on the motor rotational speed, the motor torque, the motor current and/or the time profiles thereof.

## Revendications

1. Dispositif orthopédique comprenant une partie supérieure (100) et une partie inférieure (200) qui est montée de manière déplaçable sur celle-ci et est couplée à un actionneur (30) qui déplace la partie inférieure (200) par rapport à la partie supérieure (100), l'actionneur (30) comprenant un moteur électrique (40), un dispositif de stockage d'énergie (50), un dispositif de commande (60) et un convertisseur de tension (80),
**caractérisé en ce qu'**un premier commutateur (62) est disposé entre le dispositif de stockage d'énergie (50) et le moteur électrique (40), lequel est couplé au dispositif de commande (60) et qui commute la tension d'alimentation (Us) d'une unité de commande de moteur (66) du moteur électrique (40) entre la tension de sortie (Uo) du dispositif de stockage d'énergie (50) et le convertisseur de tension (80), avec une tension d'alimentation (Us) modifiée par rapport à la tension de sortie (Uo).

2. Dispositif orthopédique selon la revendication 1,
**caractérisé en ce que** la tension de sortie du convertisseur de tension (80) est réglable de manière dynamique.

3. Dispositif orthopédique selon la revendication 1 ou 2,
**caractérisé en ce que** le convertisseur de tension (80) peut convertir aussi bien vers le haut que vers le bas ou comporte un convertisseur élévateur (82) et un convertisseur abaisseur (84).

4. Dispositif orthopédique selon la revendication 3,
**caractérisé en ce qu'**un deuxième commutateur (64) destiné à commuter entre le convertisseur élévateur (82) et le convertisseur abaisseur (84) est couplé au dispositif de commande (60).

5. Dispositif orthopédique selon l'une des revendications précédentes,
**caractérisé en ce que**, dans la position initiale du premier commutateur (62), la tension de sortie (Uo) du dispositif de stockage d'énergie (50) est appliquée comme tension d'alimentation (Us) à l'unité de commande de moteur (66) du moteur électrique (40).

6. Dispositif orthopédique selon l'une des revendications précédentes,
**caractérisé en ce que** le commutateur (62, 64) est conçu comme une partie du convertisseur de tension (80).

7. Dispositif orthopédique selon l'une des revendications précédentes,
**caractérisé en ce que** le convertisseur de tension (80) est constitué d'au moins un interrupteur à semi-conducteur, et au moins l'un de ces interrupteurs à semi-conducteur est conçu comme un commutateur (62, 64).

8. Dispositif orthopédique selon l'une des revendications précédentes,
**caractérisé en ce que** le dispositif de commande (60) est conçu de telle sorte qu'une augmentation de la tension d'alimentation (Us) de l'unité de commande de moteur (66) n'est effectuée qu'une fois qu'un seuil de vitesse de rotation du moteur électrique (40) est dépassé.

9. Dispositif orthopédique selon l'une des revendications précédentes,
**caractérisé en ce que** la tension d'alimentation (Us) de l'unité de commande de moteur (66) ou du moteur (40) dépend de la vitesse de rotation du moteur, du couple moteur, du courant moteur et/ou de leurs courbes temporelles.
